# EUROPEAN PATENT APPLICATION

(11) **EP 2 839 876 A2**
(43) Date of publication of application: **25.02.2015**
(21) Application number: 13461563.2
(22) Date of filing: 05.12.2013
(51) Int. Cl.: B01J 13/10

(54) **A method for producing microcapsules**

(30) Priority: 20.08.2013 PL 40510113
(71) Applicant: Zachodniopomorski Uniwersytet Technologiczny w Szczecinie, 70-310 Szczecin (PL)
(72) Inventor: Soból, Marcin, 71-450 Szczecin (PL); Bartkowiak, Artur, 71-667 Szczecin (PL)
(74) Representative: Zawadzka, Renata

(57) **Abstract**

A method for producing microcapsules, according to the invention, comprising of polyelectrolyte complex formation by introducing an aqueous solution of carrageenan or carrageenan hydrogel microcapsules to an aqueous solution of a cationic polymer containing quaternary amine groups, characterized in that the polymer with cationic character, containing quaternary amino groups, is poly(2-hydroxypropyl dimethyl ammonium chloride) and/or poly[oxyethylene(dimethylimino) ethylene(dimethylimino) ethylene dichloride].

## Description

The present invention provides a process for preparing microcapsules produced due to the formation of polyelectrolyte complexes, designed for the immobilization of hydrophobic compounds and hydrophilic materials of biological origin such as enzymes, cells, tissues or parts thereof.

Microencapsulation is one of the techniques that enables "closing - immobilization" of microscopic material in the core surrounded by an additional layer having specific physical and chemical properties. Nowadays microencapsulated materials are increasingly used in industry, particularly in biotechnology, where immobilization of living cells such as bacteria in the hydrogel matrix in the form of micro-bioreactors and their direct application in a variety of biotechnical processes can reduce the cost of the process. Most systems that are used during encapsulation of bacteria or cells and other tissues of organisms is based on the polyelectrolyte complex formation between alginate and divalent ions, especially calcium. Although this type of capsules did not adversely affect cell growth, they are unstable when stored in a common buffer solutions used in culture media, which is usually caused by the presence of compounds such as carbonates or phosphates, as well as monovalent metal cations. Additional coating of the alginate capsules using cationic polymers such as polylysine, polyguanidyne, polyethylenimine or chitosan is leading to relative stability improvement but only at limited level. Low stability of capsules in the culture media is a major problem standing in the way of implementation of this technology on a larger scale such as in industrial biotechnological processes. One of the most promising method that can solve this problem is to create a stable membranes around the encapsulated material by direct reaction between two polymers having opposite functional ionic groups- polyelectrolytes belonging to so-called strong polyelectrolytes (Dautzenberg H, Schuldt U, Grasnick G, Karle P, Müller P, Löhr M, Pelegrin M, Piechaczyk M, Rombs KV, Günzburg WH, Salmons B, Saller RM. Development of cellulose sulfate-based polyelectrolyte complex microcapsules for medical applications. Ann N Y AcadSci. 1999 Jun 18;875:46-63). English patent application GB 2135954 A discloses a method for producing microcapsules by a method in which the anionic polymers, used in the form of derivatives of natural polysaccharides, such as cellulose sulfate, dextran sulfate, carboxymethyl cellulose, alginate or synthetic polyelectrolytes such as poly(styrene sulfate) and cationic polymer in the form of poly(diallyldimethylammonium) are used. American invention US 4749620 shows a method of obtaining capsules by the reaction between anionic natural biopolymer - alginate, and natural cationic biopolymer chitosan. Although chitosan is being subjected to modification by implementation of new functional groups and changes in molecular weight, still such systems remain stable only in short period. The European Patent EP0188309B1 provides a process for encapsulating of mammalian cells using acrylic copolymers of anionic and cationic character resulting from the presence of functional groups such as carboxyl groups and/or primary, secondary and tertiary amine groups. In the patent description there is no information on the possibility to use carrageenan to form microcapsules. Patent PL201342 B1 discloses a process for the production of microcapsules containing a hydrogel material, with a core of hydrophobic nature, produce by the formation of polyelectrolyte complexes, characterized by a fact that its membrane is formed by dispersing a hydrophobic liquid substance (factor I) containing a ionic low-molecular weight substance (factor III) in an aqueous solution of an ionic polymer (factor II), whereas the low molecular weight substance (factor III) is not soluble in an aqueous solution of an ionic polymer (factor II), and after obtaining oil-water emulsion the aqueous solution of pH-lowering substance (factor IV) is introduced and as a result the release from the core of a gelling agent, which in these conditions is soluble in water solutions, occur. Patent 196926 discloses a process, which involves the creation of an outer membrane of the microcapsules by dropwise addition of an aqueous solution of anionic polymer (factor I) to the aqueous solution of the modified natural polymer containing low molecular weight quaternary amine (factor II). As the anionic polymer a polysaccharide having functional anionic groups and anionic proteins are used. As the modified, natural, low molecular weight polymers having quaternary amino groups, a modified polysaccharides having a molecular weight preferably in the range from 1000 g/mol to 40000 g/mol and a degree of substitution in the range of 0,1 to 3, obtained by reaction of the reactive functional groups of the polysaccharides with low molecular weight monomeric reactants containing quaternary amine groups, are applied. Carrageenans are polysaccharides containing repeating galactose units, where the basic unit of disaccharide chain consists with alternating (1,3) α-D-galactopyranose and the (1,4) β-D-galactopyranose. In some fractions of carrageenans galactopyranose is replaced with 3,6 anhydrogalactose. There are three most common fractions of carrageenan: kappa (κ), iota 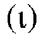 and lambda (λ) with varying degrees of substitution of sulfonic groups per repeating unit of the backbone, respectively, 0,5; 1,0; and 1,5 in the case of λ-carrageenan. Individual fractions differ in sulfonic group content and 3,6 anhydrogalactose units and therefore behave differently when creating polyelectrolyte complexes with cationic polymers, which have a direct impact on the final properties of the created capsules. Microcapsules obtained from carrageenan may be formed by the ionotropic gelation in the presence of ions, and the stability of these microcapsules is only guaranteed in case of presence of these ions in the target solution, otherwise these systems have low mechanical strength. US patent 4647536 disclose a method of producing microcapsules on the basis of carrageenan dissolved in the hot aqueous solution of NaCl and the dispersion of this aqueous solution in hydrophobic compound followed by cooling and as a result formation of hydrogel capsules.

The aim of the invention is to obtain a polyelectrolyte microcapsules formed in the two-component systems of an anionic polymer/cationic polymer solving a problem of low mechanical strength of such systems in biotechnological mediums containing compounds causing the disintegration of polyelectrolyte polyanion/polycation complexes, such as phosphates, carbonates and other salts, including salts of monovalent metals.

A method for producing microcapsules, according to the invention, comprising the polyelectrolyte complex formation by introducing an aqueous solution of carrageenan or carrageenan hydrogel microcapsules to an aqueous solution of a cationic polymer containing quaternary amine groups, characterized in a way that as the cationic polymer, containing quaternary amino groups a poly(2-hydroxypropyl dimethyl ammonium chloride) and/or poly[oxyethylene(dimethylimino) ethylene(dimethylimino) ethylene dichloride] is/are used. The cationic polymer allows the preparation of a semipermeable membrane on the outer of the carrageenan microcapsules. Polyelectrolyte complexes, forming capsules, obtained on the basis of these two ionic polymers of opposite charges, unexpectedly showed high mechanical strength and stability that far exceeds previously described in the literature systems. Creating carrageenan hydrogel microcapsules is carried out by well know methods to obtain the microcapsules, such as by dropping or ionotropic gelation or dispersing. In the process in the first variant, an aqueous solution of carrageenan is added dropwise to an aqueous solution of poly(2-hydroxypropyl dimethyl ammonium chloride) and/or poly[oxyethylene (dimethylimino) ethylene(dimethylimino) ethylene dichloride]. In another variant, an aqueous solution of κ- or τ- carrageenan is added dropwise to an aqueous solution of gelling agent in a form of metal salts, and after receiving a spherical hydrogel microcapsules, mixed with an aqueous solution of poly(2-hydroxypropyl dimethyl ammonium chloride) and/or poly[oxyethylene(dimethylimino) ethylene(dimethylimino) ethylene dichloride]. In another variant, an aqueous solution of carrageenan is dispersed in a hydrophobic compound, and after receiving a spherical hydrogel microcapsules, mixed with an aqueous solution of poly(2-hydroxypropyl dimethyl ammonium chloride) and/or poly[oxyethylene(dimethylimino) ethylene(dimethylimino) ethylene dichloride]. According to a method carrageenan having a molecular weight in the range of 50000 - 5000000 g/mol and a degree of substitution of sulfonic groups per repeating unit in the range of 0,5-3 is used. The most preferred results are obtained when one uses a carrageenan having a molecular weight in the range 200000- 800000 g/mol and a degree of substitution of sulfonic groups in the range 1-1,5. According to this method an aqueous solution of carrageenan concentration ranging from 0,1 to 10 % by weight, preferably 1-2% by weight is used. According to a method an aqueous cationic solution concentration is in the range of 0,01-10 % by weight, preferably in the range of 0,5-4% by weight. A cationic polymer having a molecular weight between 1000-1000000 g/mol, preferably between 1000-10000 g/mol is used. According to a method an aqueous solution of carrageenan and/or an aqueous solution of cationic polymer containing modifiers can be used. The addition of modifier prevents shrinkage of capsules during reaction, provides adequate osmolality of the solution, which is essential for cell encapsulation processes, changes the properties of the final microcapsules, such as mechanical strength, the size of the largest pores (so-called exclusion limit, cut-off). As modifier a nonionic polymer and/or anionic polymer and/or osmotically active substance can be used. Additives affect the strength, the structure of the pores in the capsule, its stability. With longer reaction times modifier prevents shrinkage of microcapsules. The preferred nonionic polymers used contain dextran and/or polyvinyl alcohol and/or methylcellulose and/or modified starch (sodium octenyl succinate starch). Preferably the anionic polymers used contain alginate and/or cellulose sulfate, and/or dextran sulfate. This modifier affects the strength of the microcapsules and pore size. Preferably, the osmotically active substances used contain glucose and/or mannitol and/or HEPES (2-[4-(2-hydroxyethyl)-1-piperazinyl] ethanesulfonic acid), compounds not causing gelation of a carrageenan solution. The addition of these substances avoid osmotic shock which may be experience by bacteria during the immobilization. It also allows to change the size of the pores, the strength, the structure of the membrane. As a metal salts, monovalent salts, such as potassium chloride and divalent such as calcium chloride are used. As the hydrophobic compound soybean oil and/or paraffin oil and/or canola oil are used.

The advantage of microcapsules produced according to the invention is its high initial mechanical strength, which is maintained in spite of storage in medium solutions, which reduce the strength or even disintegration of microcapsules described so far in the literature. An additional advantage is that the capsules have a semipermeable membrane. Microcapsules according to the invention are characterized by improved mechanical strength and high stability during storage in buffers and biotechnological mediums containing polyvalent salts. Microcapsules obtained by the proposed method achieved significantly higher mechanical resistance to compression of up to more than 15N per capsule. Additionally, utilization of additive in the form of a non-ionic polymer to the natural anionic polyelectrolyte, not affecting the properties of the immobilized biological material, solves the problem of capsule shrinkage during the complexation reaction. This solution allows to obtain microcapsules with high stability during storage in mediums used in biotechnological processes including body fluids and solutions. Equally important, when placed in a nutrient solution used for example in biotechnology bioconversion of wasted glycerol to polyols and dicarboxylic acids containing in its composition K₂HPO₄ (3,8167 g/dm³), KH₂PO₄ (3 g/dm³) and other salts, capsules retain their high strength. Even in case of multiple capsules transferring to new medium mechanical strength is maintained at a level of 13,4 - 17,5 N, which enables a 4-fold passage of microcapsules during 28 days of culture. Phosphates present in the medium do not cause disintegration of the formed membrane, which ensures the integrity of the capsules. For comparison, the mechanical strength of widely used alginate/calcium capsules is 6N and decreases to 0,1 N after 7 days of storage in said medium. The value of so-called cut-off, the size of the largest pores present in the membrane of the microcapsules, prepared according to the above method, can be adjusted depending upon the concentration of polymer, the reaction time and the addition of low molecular weight compounds both to the solution of an anionic polymer and a cationic polymer. For the system 2% by weight 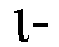 carrageenan + 3% by weight dextran/ 0,5% by weight poly(2-hydroxypropyl dimethyl ammonium chloride), and reaction time 30 minutes the resulting capsules have a cut-off value at 90000 g/mol. When using HEPES additive to polycation in an amount of 3,67% by weight an increased in the cut-off of membrane to the level of 180000 g/mol can be observed. The extension of the reaction time to 60 minutes led to decrease of cut-off to 70000 g/mol and 150000 g/mol, respectively. The value of cut- off is particularly important in case of using the described system for the immobilization of living cells to produce systems with a defined pore size, e.g. bioimmobilisation for a purpose of transplantation to received so-called bioartificial organs. Membrane cut-off at the appropriate level will assure diffusion of nutrients into the capsule on one hand and prevent penetration of antibodies against cells subjected to encapsulation on the other.

According to the method the polyanion solution used is a carrageenan, which is a polysaccharide of natural origin, which means that it has a higher biocompatibility compared to other polyanions used in the encapsulation process, such as cellulose sulfate or dextran sulfate, obtained by chemical modification, sulfonation, of natural polysaccharides cellulose and dextran, respectively.

The invention is further illustrated in the following examples.

### Example I

In 9,8 cm³ of distilled water 0,2 g of 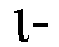 carrageenan was dissolved under vigorous stirring with a magnetic and/or mechanical stirrer. After thorough dissolution lasting approximately 6-24 hours, the solution manually or using a syringe pump was added dropwise using a syringe with a capacity of 10 cm³ and a needle with a diameter of 0,7 mm to 100 cm³ of a stirred aqueous solution of 1% by weight poly(2-hydroxypropyl dimethyl ammonium chloride) with the formula (C₅H₁₂ClNO)ₙ. On the carrageenan drop surface a thin polyelectrolyte membrane is formed which ensures the integrity of capsules during the subsequent process of membrane formation. After 15 minutes of complexation capsules, size with a range of 2-3 mm, are removed by decantation from the polycation solution and then washed 3-fold with distilled water.

### Example II

A method performed as described in Example I, except that the concentration of poly(2-hydroxypropyl dimethyl ammonium chloride) is 10% by weight and complexation time is 60 minutes. The resulting capsules are completely shrunk, but they return to a spherical shape after three-fold washing with 200cm³ of distilled water and then stirring for at least one hour in 100 cm³ of distilled water. The resulting capsules have a compressive strength of 15N. After placing the capsules in 100cm³ of nutrient solution having in its composition: glycerol (20 g/dm³), K₂HPO₄ (3,8167 g/dm³), KH₂PO₄ (3 g/dm³), as well as other salts such as (NH₄)₂SO₄, MgSO₄*7H₂O, CaCl₂*2H₂O, CoCl₂*6H₂O and yeast extract, bacteriological peptone, bacteriological meat extract, capsules retain their high mechanical strength by 28 days, even at weekly medium change to a new one.

### Example III

Microcapsules prepared similarly to example I, except that instead of τ-carrageenan a λ-carrageenan fraction is used and cationic polymer poly[oxyethylene(dimethylimino) ethylene(dimethylimino) ethylene dichloride] of formula (C₁₀H₂₄Cl₂N₂O)ₙ is used.

### Example IV

Microcapsules prepared similarly to example I, except that instead of 1% poly(2-hydroxypropyl dimethyl ammonium chloride) a 1% by weight aqueous solution, being a 1:1 weight mixture of poly(2-hydroxypropyl dimethyl ammonium chloride) and poly[oxyethylene(dimethylimino) ethylene(dimethylimino) ethylene dichloride], is used.

### Example V

In 9,8 cm³ of distilled water 0,2 g of κ-carrageenan, with a degree of substitution of 0,5 of sulfate groups on the repeating unit of the backbone, is dissolved under vigorous stirring with a magnetic stirrer. After thorough dissolution lasting approximately 6-24 hours, the solution manually or using a syringe pump is added dropwise using a syringe with a capacity of 10 cm³ and a needle with a diameter of 0,7 mm to 100 cm³ stirred aqueous solution of 0,3M potassium chloride. After 30 minutes of reaction capsules having a size of 2-3 mm are removed by decantation and washed 3-times with distilled water. Then hydrogel capsules are placed in a 0,01% by weight aqueous solution of poly(2-hydroxypropyl dimethyl ammonium chloride) having a molecular mass of 500000 g/mol. After 45 minutes capsules have an outer polymer membrane formed in a complexation reaction. Next capsules are removed by decantation from the polycation solution and washed 3-times with distilled water.

### Example VI

Similarly to example V, except that instead of κ-carrageenan a 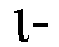 carrageenan and instead of potassium chloride a calcium chloride are used and the molecular weight of poly(2-hydroxypropyl dimethyl ammonium chloride) is 1000000 g/mol.

### Example VII

In 8,1 cm³ of distilled water 0,2 g of 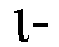 carrageenan, with a degree of substitution of sulfate groups on the repeating unit of the backbone equal to 1, and a molecular weight of 800000 g/mol, 0,3g of dextran, having a molecular weight >1000000g/mol and 0,4 g of glucose are dissolved. The resulting solution has an osmolality of 290 mOsmol/kg, which is the optimum value for *Citrobacter freundii,* a bacteria carrying out the process of bioconversion of waste glycerol. In order to sterilize, the solution is filtered, first using a filter paper and then using a polyethersulfone (PES) membrane, wherein the filter pore size is <0,22 mm. To so-prepared sterile solution 1 ml of bacteria, having a concentration of 5 in McFarland scale, is added. After mixing the solution is added dropwise , using gravitational forces to 100 cm³ of a stirred 1% poly(2-hydroxypropyl dimethyl ammonium chloride) having a molecular weight of about 1800 g/mol with an additive of 4,6g of glucose, which ensure osmolality of the solution at a level of 290 mOsmol/kg. Complexation time is 30 minutes. For the resulting capsules concentration of the bacteria in capsules is 2,5*10³cfu/capsule. After 72 hours of culture, this value is increased to 4,8*10⁷cfu/capsule. After this time, capsules are 3 times moved to new medium, when performing 24-hour culture. An increase in the concentration of bacteria to 8,7*10⁷ cfu/capsule can be observed. During the culture a protrusion of bacteria from the capsule inside to the medium can be observed, at a level of 1,9*10⁸cfu/ml after 72 hours which is equal to 8,5% of similarly conducted free culture. The protrusion is decreasing to 1,1*10⁸-5,5*10⁷cfu/ml and a downward trend can be observed.

### Example VIII

Microcapsules are prepared similarly to example VII, but instead of 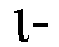 carrageenan a λ-carrageenan, with a degree of substitution of sulfate groups on the repeating unit of the backbone equal to 1,5 and a molecular mass of 1200000 g/mol, is used and instead of poly(2-hydroxypropyl dimethyl ammonium chloride) a poly[oxyethylene(dimethylimino) ethylene(dimethylimino) ethylene dichloride] is used.

### Example IX

In 9,0 cm³ of distilled water at 80°C 1g of 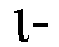 carrageenan, having a molecular mass of 50000 g/mol, is dissolved with vigorous stirring with a mechanical stirrer. Than the solution is dispersed in 50 cm³ of rapeseed oil until the carrageenan microspheres with the desired diameter are formed, after which the mixture is cooled to 5°C. Collected 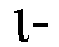 carrageenan spheres are washed 3-times with water, and then on their surface an outer membrane is formed through the introduction and mixing for 15 minutes in a 0,5% by weight aqueous solution of poly(2-hydroxypropyl dimethyl ammonium chloride). After the reaction capsules are washed 3-times with distilled water.

### Example X

Similarly to example IX, but instead of 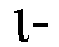 carrageenan a 0,1g of λ-carrageenan with molar mass of 5000000 g/mol is used.

### Example XI

In 9,55 cm³ of distilled water 0,15 g of 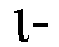 carrageenan and 0,3 g of dextran, having a molecular mass >1000000 g/mol, is dissolved under vigorous stirring with a magnetic and/or mechanical stirrer. After thorough dissolution lasting approximately 6-24 hours, the solution is manually or using a syringe pump added dropwise using a syringe with a capacity of 10 cm³ and a needle with a diameter of 0,7 mm to 100 cm³ of a mixed aqueous solution of 0,5% by weight poly(2-hydroxypropyl dimethyl ammonium chloride) and 3,67% by weight 2-[4-(2-hydroxyethyl)-1-piperazinyl]ethanesulfonic acid. After 30 minutes of complexation capsule with a size range of 2-3 mm are removed by decantation from the polycation and then washed 3-times with distilled water. Capsules have an upper exclusion limit (cut-off) equal to 70000 g/mol.

### Example XII

Similarly to example XI, but the concentration by weight of dextran is being increased to 5%. This led to increased of cut-off to 120000 g/mol.

### Example XIII

Similarly to example XII, but the dripped solution contain addition of 3,67% by weight 2-[4-(2-hydroxyethyl)-1-piperazinyl]ethanesulfonic acid. As a result cut-off is increased to 150000 g/mol.

### Example XIV

Similarly to example XII, but instead of dextran a sodium salt of octenylsuccinate starch is used. The resulting capsules have a cut-off equal to 60000 g/mol.

### Example XV

Similarly to example I, but instead of 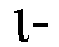 carrageenan a 1:1 weight mixture with dextran sulfate is used.

### Example XVI

Similarly to example VI, but instead of 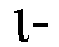 carrageenan a 1:1 weight mixture with sodium alginate is used.

### Example XVII

Similarly to example IX, but instead of 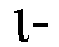 carrageenan a 1:1 weight mixture with mannitol is used.

### Example XVIII

Similarly to example I, but instead of τ-carrageenan a κ-carrageenan is used.

### Example XIX

Similarly to example IX, but instead of 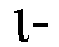 carrageenana a κ-carrageenan is used.

## Claims

1. A method for producing microcapsules comprising of polyelectrolyte complex formation by introducing an aqueous solution of carrageenan or carrageenan hydrogel microcapsules to an aqueous solution of a cationic polymer containing quaternary amine groups, **characterized in that** the polymer with cationic character, containing quaternary amino groups, is poly(2-hydroxypropyl dimethyl ammonium chloride) and/or poly[oxyethylene(dimethylimino) ethylene(dimethylimino) ethylene dichloride].

2. A method according to claim 1, **characterized in that** the aqueous carrageenan solution is added dropwise to a aqueous solution of poly(2-hydroxypropyl dimethyl ammonium chloride) and/or poly[oxyethylene(dimethylimino) ethylene(dimethylimino) ethylene dichloride].

3. A method according to claim 1, **characterized in that** the aqueous solution of κ- or τ-carrageenan is added dropwise to a aqueous gelling metals salts solution, and after obtaining spherical hydrogel microcapsules, are mixed with aqueous solution of poly(2-hydroxypropyl dimethyl ammonium chloride) and/or poly[oxyethylene(dimethylimino) ethylene(dimethylimino) ethylene dichloride].

4. A method according to claim 1, **characterized in that** the aqueous carrageenan solution is dispersed in hydrophobic compound, and after forming spherical hydrogel microcapsules, are mixed with aqueous solution of poly(2-hydroxypropyl dimethyl ammonium chloride) and/or poly[oxyethylene(dimethylimino) ethylene(dimethylimino) ethylene dichloride].

5. A method according to claim 1, **characterized in that** the carrageenan used have a molar mass of 50000- 5000000 g/mol and a degree of substitution of sulfate groups on the repeating unit of the backbone in a range of 0,5-3.

6. A method according to claim 1, **characterized in that** an aqueous solution have a carrageenan concentration in a range of 0,1- 10% by weight.

7. A method according to claim 1, **characterized in that** an aqueous solution have a polycation concentration in a range of 0,01- 10% by weight.

8. A method according to claim 1, **characterized in that** a polymer with a cationic character have a molar mass in a range of 1000- 1000000 g/mol.

9. A method according to claim 1, **characterized in that** an aqueous solution of carrageenan and/or aqueous solution of polymer with cationic character have an addition of modifier.

10. A method according to claim 9, **characterized in that** as modifier a nonionic and/or an anionic polymer and/or an osmotic active compound is/are used.

11. A method according to claim 10, **characterized in that** as nonionic polymer dextran and/or poly(vinyl alcohol) and/or methylcellulose and/or modified starch is/are used.

12. A method according to claim 10, **characterized in that** as anionic polymer an alginate and/or cellulose sulfate and/or dextran sulfate is/are used.

13. A method according to claim 10, **characterized in that** as osmotic active compound a glucose and/or mannitol and/or 2-[4-(2-hydroxyethyl)-1-piperazinyl]ethanesulfonic acid is/are used.

14. A method according to claim 3, **characterized in that** as metal salts potassium chloride and/or calcium chloride is/are used.

15. A method according to claim 4, **characterized in that** as hydrophobic compound a soybean oil and/or paraffin oil and/or canola oil is/are used.
